# EUROPEAN PATENT SPECIFICATION

(11) **EP 3 928 686 B1**
(45) Date of publication and mention of the grant of the patent: **03.07.2024**
(21) Application number: 20758724.7
(22) Date of filing: 19.02.2020
(51) Int. Cl.: A61C 9/00, A61C 13/00, A61B 5/00

(54) **COMBINED INTRAORAL AND IMPRESSION SCANNING SYSTEM AND METHOD**
SYSTEM UND VERFAHREN FÜR KOMBINIERTE INTRAORALE UND ABDRUCKSCANS
SYSTÈME ET PROCÉDÉ DE BALAYAGE D'IMPRESSION ET INTRAORAL COMBINÉ

(30) Priority: 19.02.2019 KR 20190019296
(43) Date of publication of application: 29.12.2021
(73) Proprietor: Medit Corp., Seoul 02855 (KR)
(72) Inventor: LEE, Dong Hoon, Goyang-si Gyeonggi-do 03912 (KR); CHOI, Won Hoon, Seoul 05823 (KR); SUH, Beom Sik, Seoul 03301 (KR)
(74) Representative: Klunker IP Patentanwälte PartG mbB
(86) International application number: PCT/KR2020/002391
(87) International publication number: WO 2020/171589

(56) References cited:
- WO-A1-2012/083960
- WO-A1-2012/083960
- JP-A- 2009 517 144
- JP-B2- S6 262 179
- KR-A- 20170 113 412
- KR-B1- 101 340 971

## Description

### [Technical Field]

The present disclosure relates to a combined oral and impression scanning system and method, and more particularly, to a combined oral and impression scanning system and method for obtaining one final digital datum merged by aligning impression scan data with oral scan data in a process of obtaining the impression scan data after obtaining the oral scan data by using an oral scanner.

### [Background Art]

In general, three-dimensional (3-D) scanning is used in various industry fields, such as reverse engineering, measurement, tests, content creation, and a CAD/CAM. With the development of the computing technology, scanning performance is improved and fields in which scanning is used are gradually expanded.

Recently, a real-time scanning technology which can obtain data having several frames or more per second and allows a common user who has not experienced professional training to easily obtain 3-D data through automated data processing is improved by leaps and bounds.

In this case, for real-time scanning, a structured light method of projecting a structured pattern onto an object to be measured by using an optical projection device, obtaining an image of the object through an imaging device, and computing 3-D data by analyzing the obtained image is chiefly used.

The reason for this is that such a structured light method is suitable to be used in real-time scanning for measuring 3-D data while moving a scanner at a high speed because 3-D data for a wide area can be obtained at a time.

However, although the number of patterns is sufficiently reduced, a location relation between an object to be measured and the scanner is slightly changed whenever each of the patterns is photographed as the object to be measured moves upon real-time measurement. Accordingly, in the case of many real-time scanning patterns, a design is planned to be robust against such a change to some extent. Nevertheless, there is a problem in that data quality and the amount of data obtained are reduced due to a movement.
WO 2012/083960 A1 discloses a system and a method of scanning an object being modified comprising the steps of: providing a first 3D representation of the object prior to modification, obtaining at least a second 3D representation by scanning at least a modified part of the object after modification, aligning said first and second 3D representations, and replacing a first subset of the first 3D representation with data from the second 3D representation, said first subset substantially corresponding to the modified part, thereby updating the first 3D representation to represent the modified object.

### [DISCLOSURE]

### [Technical Problem]

An object of the present disclosure is to provide a combined oral and impression scanning system and method, which enable elaborate prosthetics to be fabricated using obtained digital data by obtaining one final scan datum merged by aligning impression scan data with oral scan data in a process of obtaining the impression scan data after obtaining the oral scan data by using an oral scanner.

Technical objects of the present disclosure are not limited to the aforementioned technical object, and other technical objects not described above may be evidently understood by those skilled in the art from the following description.

### [Technical Solution]

A combined oral and impression scanning system according to the present disclosure may include an oral scanner configured to scan an oral object, a storage unit configured to store oral scan data obtained from the oral scanner and selectively store impression scan data of the oral object according to a preset determination condition, a determination unit configured to determine whether the oral scan data corresponds to the determination condition and guide selective storage of the impression scan data, and a control unit configured to control a selection area corresponding to the determination condition, among the impression scan data, to be partially applied to a corresponding area of the oral scan data.

Furthermore, the determination condition may include at least one of a plurality of conditions, including the unclear indication of a margin line, the presence of a post area or the presence of an area including metal in the oral scan data.

Furthermore, the control unit may include a simulation unit configured to receive, from the storage unit, information on areas neighboring the selection area and to automatically simulate the selection area before the selection area is applied to be replaced.

Meanwhile, a combined oral and impression scanning method according to the present disclosure may include an oral scan data storage step of storing oral scan data by scanning an oral object, an impression mode change step of changing a mode of the oral scan data into an impression mode so that the oral scan data corresponds to selectively stored impression scan data, a selection area replacement application step of partially obtaining scan data of a selection area among the impression scan data and applying the obtained scan data to the oral scan data having the changed impression mode, and a final scan datum acquisition step of changing again, into an oral mode which is an initial mode, the impression mode of the oral scan data to which the impression scan data has been applied in the selection area and obtaining the final scan data.

Furthermore, the method may further include a prosthetics production step of transmitting the final data to a design program so that prosthetics is fabricated.

Furthermore, the oral object may include an oral model or an inside of a mouth of a patient.

Furthermore, the method may further include an impression scan data storage determination step of selectively storing the impression scan data depending on whether the oral scan data includes a preset determination condition after the oral scan data storage step.

Furthermore, the determination condition may include at least one of a plurality of conditions, including the unclear indication of a margin line, the presence of a post area or the presence of an area including metal in the oral scan data.

Furthermore, the impression scan data may include data stored by selectively scanning only a portion corresponding to a portion of oral scan data corresponding to the determination condition in an impression model.

Furthermore, the impression mode change step may include indicating a margin line in the impression scan data by using an impression material when it is determined that the indication of the margin line is unclear in the oral scan data in the oral scan data storage step. The margin line may be applied to the oral scan data and indicated when the final scan data is obtained.

Furthermore, the oral scan data storage step may include a post area scan step of converting a mode of the post area into an impression mode when it is determined that the selection area is a post area by scanning the oral object and applying the selection area by replacing previously stored impression scan data.

Furthermore, the post area scan step may include a display step of displaying the post area, an impression mode change step of selectively obtaining oral scan data of the post area, converting a mode of the oral scan data into the impression mode, and obtaining impression scan data, and an embossing scan data application step of obtaining embossing scan data of the post area by changing the impression mode and applying the embossing scan data to the impression scan data by partially replacing the impression scan data.

Meanwhile, a combined oral and impression scanning method according to another embodiment of the present disclosure may include an oral scan data acquisition step of obtaining, by a scanner, oral scan data of an oral object including a post area, an impression mode change step of changing a mode of the oral scan data into an impression mode so that the oral scan data is reversed, a selection area designation step of designating the post area as an object area for an impression scan, an impression scan data acquisition step of obtaining impression scan data of the selection area designated as the object area for the impression scan, and a data aligned step of aligning the oral scan data and the obtained impression scan data of the selection area.

Furthermore, the impression scan is obtained by scanning an impression model which is a frame of an oral model through a scanner.

Furthermore, in the impression scan data acquisition step, the post area may be obtained in an embossing form.

Furthermore, the method may further include a re-reverse step of reversing again the oral scan data and impression scan data aligned in the data aligned step when the impression mode is released.

Furthermore, the method may further include a final scan datum acquisition step of replacing the oral scan data with the impression scan data of the selection area in the re-reverse step and obtaining final scan data by combining the oral scan data and the impression scan data.

Meanwhile, a combined oral and impression scanning method according to still another embodiment of the present disclosure may include a first oral scan data storage step of storing first oral scan data by scanning an outer surface of an oral object including an upper jaw model and a lower jaw model, a second oral scan data storage step of storing second oral scan data by scanning an inside of the oral object in a state in which the upper jaw model and the lower jaw model have been isolated from each other, a selection area impression scan data comparison step of obtaining impression scan data of a selection area of the oral object including an occlusion state of the upper jaw model and the lower jaw model and comparing the first oral scan data and the second oral scan data, and a selection area impression scan data replacement application step of applying the impression scan data to the first oral scan data and the second oral scan data by partially replacing the first oral scan data and the second oral scan data.

Furthermore, in the first oral scan data storage step, the upper jaw model and the lower jaw model may face each other in a way to be occluded.

### [Advantageous Effects]

The present disclosure has an effect in that elaborate prosthetics can be fabricated using obtained digital data in a way to obtain one final scan datum merged by aligning impression scan data with oral scan data in real time in a process of obtaining the impression scan data after obtaining the oral scan data by using the oral scanner.

Furthermore, the present disclosure has an effect in that more accurate digital data can be obtained by expressing a margin line in a process of obtaining an impression when it is difficult to measure the margin line because the margin line is covered with a gum (gingival) tissue in an environment within a mouth.

Meanwhile, the present disclosure has an effect in that it can solve a conventional problem in which it is difficult or impossible to obtain data by using the oral scanner due to the depth of a post areahaving an engraved form, in such a way to enable a portion whose scanning is impossible to be scanned by obtaining, in an embossing form, the post area actually having the engraved form by obtaining impression scan data.

Furthermore, the present disclosure has an effect in that a teeth portion whose accurate scanning is difficult due to a material characteristic can be scanned by separately obtaining an impression of the teeth portion covered with a crown made of metal and using such impression scan data for occlusion and alignment necessary to obtain the final scan data.

### [Description of Drawings]

FIG. 1 is a diagram schematically illustrating a combined oral and impression scanning system according to the present disclosure.
FIG. 2 is a flowchart sequentially illustrating a combined oral and impression scanning method according to a first embodiment of the present disclosure.
FIG. 3 is a diagram illustrating the acquisition of oral scan data and impression scan data in the combined oral and impression scanning method according to the first embodiment of the present disclosure.
FIG. 4 is a diagram illustrating a change into an impression mode in the combined oral and impression scanning method according to the first embodiment of the present disclosure.
FIG. 5 is a diagram illustrating a selection area in the combined oral and impression scanning method according to the first embodiment of the present disclosure.
FIG. 6 is a diagram illustrating the replacement of oral scan data with impression scan data in a selection area in the combined oral and impression scanning method according to the first embodiment of the present disclosure.
FIG. 7 is a diagram illustrating the final scan data in the combined oral and impression scanning method according to the first embodiment of the present disclosure.
FIG. 8 is a diagram illustrating the indication of a margin line in a combined oral and impression scanning method according to a second embodiment of the present disclosure.
FIG. 9 is a diagram illustrating the replacement and application of a margin line in the combined oral and impression scanning method according to the second embodiment of the present disclosure.
FIG. 10 is a diagram illustrating a post area in a combined oral and impression scanning method according to a third embodiment of the present disclosure.
FIG. 11 is a diagram illustrating the state in which a selection area for the post area is indicated in the combined oral and impression scanning method according to the third embodiment of the present disclosure.
FIG. 12 is a diagram illustrating that embossing scan data is applied to the post area in the combined oral and impression scanning method according to the third embodiment of the present disclosure.
FIG. 13 is a diagram sequentially illustrating a combined oral and impression scanning method according to a fourth embodiment of the present disclosure.
FIG. 14 is a diagram illustrating a post area in the combined oral and impression scanning method according to the fourth embodiment of the present disclosure.
FIG. 15 is a diagram illustrating the state in which a selection area for the post area is indicated in the combined oral and impression scanning method according to the fourth embodiment of the present disclosure.
FIG. 16 is a diagram illustrating that embossing scan data is applied to the post area in the combined oral and impression scanning method according to the fourth embodiment of the present disclosure.
FIG. 17 is a diagram illustrating the final scan data obtained using the combined oral and impression scanning method according to the fourth embodiment of the present disclosure.
FIG. 18 is a diagram sequentially illustrating a combined oral and impression scanning method according to a fifth embodiment of the present disclosure.
FIG. 19 is a diagram illustrating the state in which an upper jaw and a lower jaw face each other in the combined oral and impression scanning method according to the fifth embodiment of the present disclosure.
FIG. 20 is a diagram illustrating a covered state of an impression material in the combined oral and impression scanning method according to the fifth embodiment of the present disclosure.

### [Description of Reference Numerals]

- 1:: upper jaw 2: lower jaw
- 3:: impression
- 10:: oral scan data 10': reverse data of oral scan data
- 12:: scan area 20: impression scan data
- 20':: reverse data of impression scan data 22: scan screen
- 30:: final scan datum 42: impression mode change button
- 44:: area selection button
- 100:: oral scanner 200: storage unit
- 300:: determination unit 400: control unit
- 410:: simulation unit
- A:: selection area M: margin line
- P:: post area

### [Best Mode]

Hereinafter, some embodiments of the present disclosure will be described in detail with reference to exemplary drawings. In adding reference numerals to the elements of each drawing, it should be noted that the same elements have the same reference numerals as much as possible even if they are displayed in different drawings. Furthermore, in describing embodiments of the present disclosure, when it is determined that a detailed description of the related well-known configuration or function hinders understanding of an embodiment of the present disclosure, the detailed description thereof will be omitted.

Furthermore, in describing elements of an embodiment of the present disclosure, terms, such as a first, a second, A, B, (a), and (b), may be used. Such terms are used only to distinguish one component from the other component, and the essence, order, or sequence of a corresponding component is not limited by the terms. All terms used herein, including technical or scientific terms, have the same meanings as those commonly understood by a person having ordinary knowledge in the art to which an embodiment pertains, unless defined otherwise in the specification. Terms, such as those commonly used and defined in dictionaries, should be construed as having the same meanings as those in the context of a related technology, and are not construed as being ideal or excessively formal unless explicitly defined otherwise in the specification.

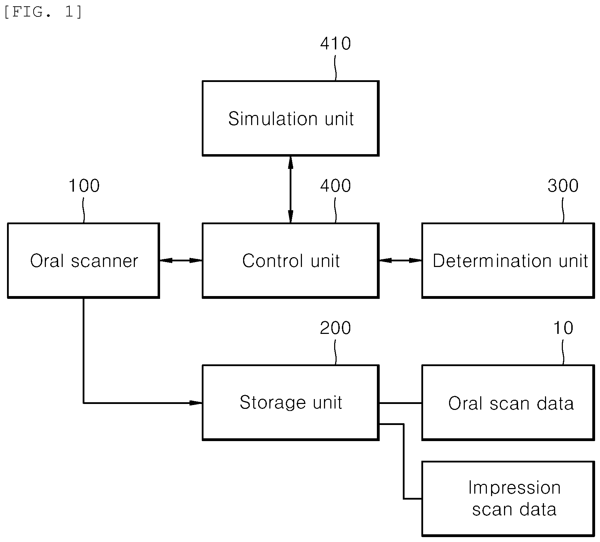

FIG. 1 is a diagram schematically illustrating a combined oral and impression scanning system according to the present disclosure. Referring to FIG. 1, the combined oral and impression scanning system according to the present disclosure includes an oral scanner 100, a storage unit 200, a determination unit 300 and a control unit 400.

First, the oral scanner 100 may correspond to any one of a model scanner having a table form or a three-dimensional (3-D) scanner having a handheld form, for photographing an oral object (in the present disclosure, an oral object is a frame whose impression has been taken or an oral model obtained by pouring plaster into a frame whose impression has been taken, or may be the inside of the mouth of an actual patient). The oral scanner 100 may include photographing means for photographing a movement. The storage unit 200 is formed to store oral scan data obtained by the oral scanner 100 and to also selectively store impression scan data of the oral object according to a preset determination condition. That is, the storage unit 200 stores the oral scan data, and also stores the impression scan data of the oral object when the determination unit 300 determines that the stored scan data corresponds to the preset determination condition. That is, the determination unit 300 determines whether the oral scan data corresponds to the preset determination condition. The selective storage of the impression scan data in the storage unit 200 may be guided based on a result of the determination of the determination unit 300.

Meanwhile, the determination condition includes at least one of a plurality of conditions, including that the indication of a margin line is not clearly indicated in the oral scan data because the indication of the margin line is covered with gums, etc., a post area P is present or an area including a part made of metal, etc. is present. Such a portion is set as a selection area. More specifically, a surface of the oral object is scanned using the oral scan data. If a margin line whose measurement is difficult because the margin line is covered with a gum tissue in an environment within a mouth, a post area having an engraved portion, or a metal area filled with amalgam or resin is present, accurate scan data for such a portion cannot be secured. Accordingly, if at least one of a plurality of such determination conditions is included, the determination unit 300 enables the impression scan data of the oral object including the selection area to be scanned by the oral scanner 100 and to be selectively stored in the storage unit 200.

Furthermore, when the oral scan data corresponds to any one of the plurality of determination conditions, the control unit 400 determines that accurate final scan data of the oral object cannot be obtained because the margin line, the post area, the metal area, etc. is present, and controls a selection area, including impression scan data and corresponding to the determination condition, to be partially applied to the oral scan data. In this case, to partially apply the impression scan data to the oral scan data may mean that data values of the oral scan data formed in the selection area are merged by performing a process of supplementing or replacing the data values of the oral scan data based on data values of the impression scan data. Preferably, to partially apply the impression scan data to the oral scan data may mean that the impression scan data replaces the oral scan data with respect to the selection area.

Accordingly, the control unit 400 controls not only the oral scan data, but the impression scan data to be selectively obtained independently and to be stored in the storage unit 200, and controls the stored data to be divided into a portion to be retained in the oral scan data and a portion to be replaced with the impression scan data, that is, as a selection area and to be partially aligned and merged. When scan data is obtained by the oral scanner 100, a portion not clearly represented based on only oral scan data can be selectively represented in the same level as an actual level. As a result, accurate final scan datum which may be used in a design task for an oral object can be effectively secured.

Meanwhile, the control unit 400 may be connected to a simulation unit 410, and may share information with the simulation unit 410. The simulation unit 410 is formed to receive, from the storage unit 200, information on areas neighboring a selection area, that is, portions before/after the selection area, in addition to the selection area included in the impression scan data and to automatically simulate the selection area before oral scan data is applied by replacing the selection area. Accordingly, the simulation unit 410 can prevent a problem in that a selection area is erroneously replaced with a wrong area in a process of automatically replacing the selection area with a corresponding area of oral scan data. As a result, a corresponding area can be accurately applied by replacing the selection area.

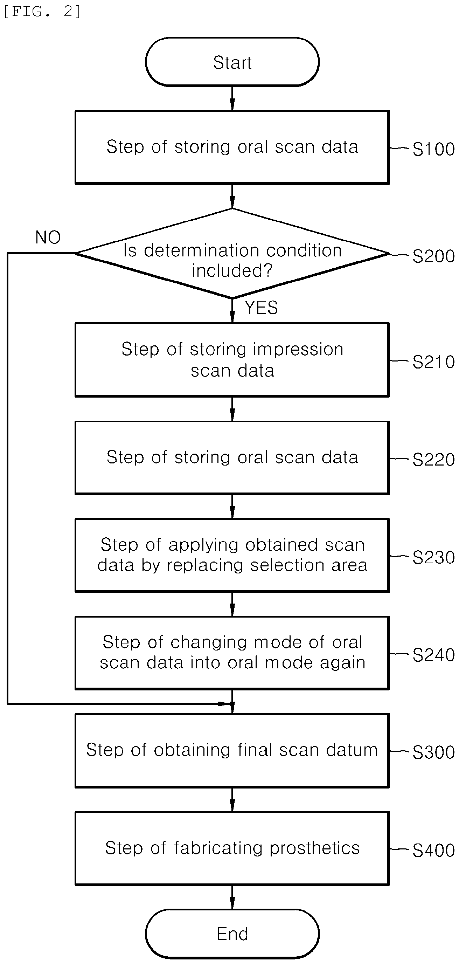

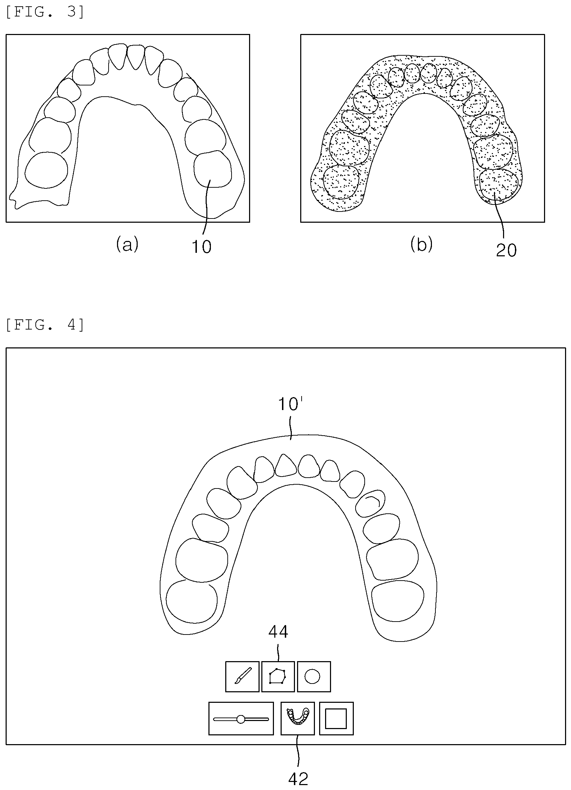

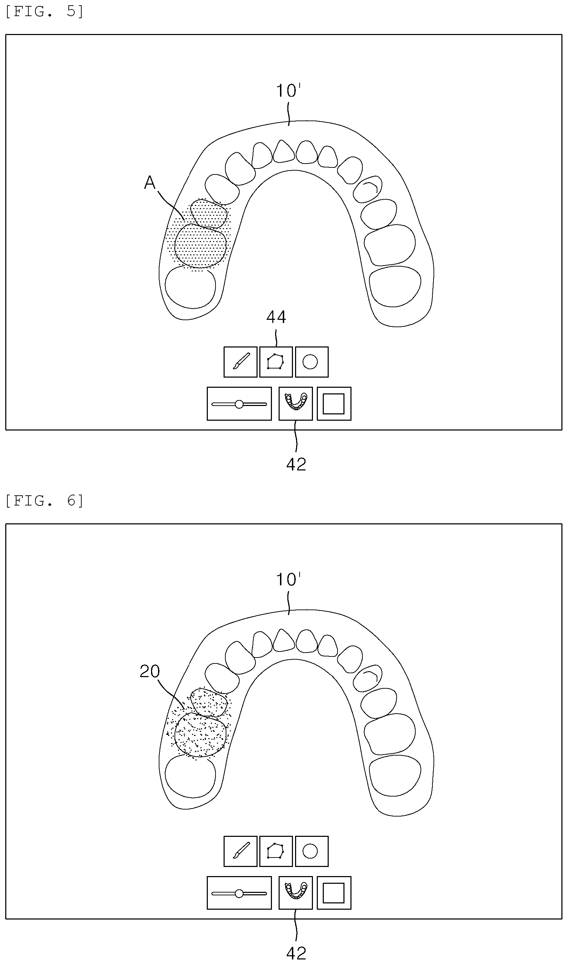

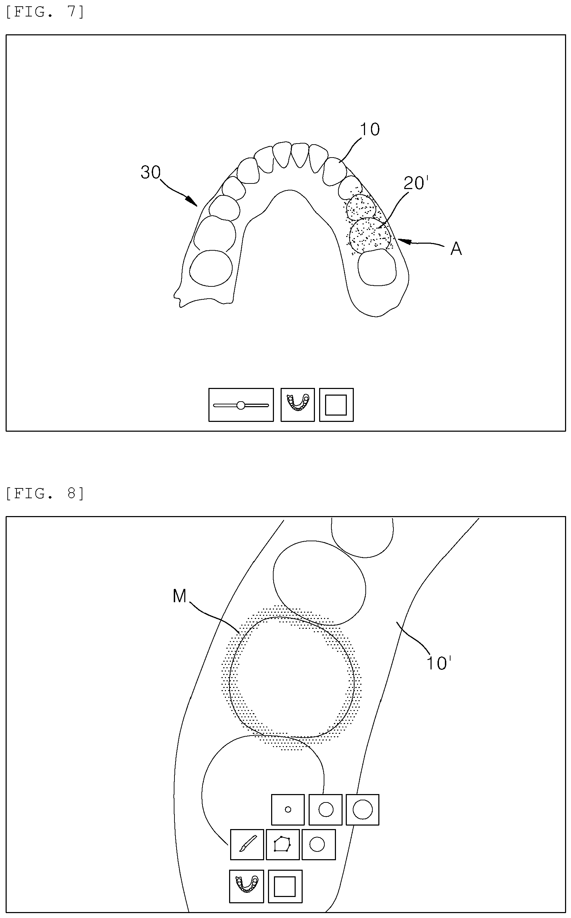

FIG. 2 is a flowchart sequentially illustrating a combined oral and impression scanning method according to a first embodiment of the present disclosure. FIG. 3 is a diagram illustrating the acquisition of oral scan data and impression scan data in the combined oral and impression scanning method according to the first embodiment of the present disclosure. FIG. 4 is a diagram illustrating a change into an impression mode in the combined oral and impression scanning method according to the first embodiment of the present disclosure. FIG. 5 is a diagram illustrating a selection area in the combined oral and impression scanning method according to the first embodiment of the present disclosure. FIG. 6 is a diagram illustrating the replacement of oral scan data with impression scan data in a selection area in the combined oral and impression scanning method according to the first embodiment of the present disclosure. FIG. 7 is a diagram illustrating the final scan data in the combined oral and impression scanning method according to the first embodiment of the present disclosure.

As illustrated in FIG. 2, the combined oral and impression scanning method according to the present embodiment is sequentially described as follows.

First, as illustrated in FIG. 3, an oral object is scanned using the oral scanner 100, and corresponding oral scan data 10 is stored in the storage unit 200 (S100). In this case, the determination unit 300 determines whether an area corresponding to a preset determination condition is present by checking the oral scan data 10 (S200), and also enables impression scan data 20 of the oral object to be selectively stored (S210). That is, as the oral scan data 10 is stored in the storage unit 200, when it is determined that an area corresponding to the preset determination condition is present by checking the stored oral scan data 10 (S200), the impression scan data 20 of the oral object is selectively stored (S210). Meanwhile, as described above, the oral object may be a frame whose impression has been taken or may be an oral model obtained by pouring plaster into a frame whose impression has been taken. Alternatively, the oral object may be the inside of the mouth of an actual patient.

Meanwhile, the determination condition includes at least one of a plurality of conditions, including that the indication of a margin line in the oral scan data 10 is covered (the unclear indication of the margin line), a post area is present or an area including metal is present. Such a portion is set as a selection area. If it is confirmed that an area corresponds to the determination condition is not present by checking the oral scan data 10, the process is terminated by considering the oral scan data 10 to be the final scan datum 30 (S300).

Meanwhile, when the impression scan data 20 of the oral object is stored (S210), it is determined that an area corresponding to the determination condition is present in the oral scan data 10. A mode of the oral scan data 10 is changed into an impression mode so that the oral scan data 10 corresponding to the selectively stored impression scan data 20 as described above (S220). In other words, in order to convert the oral scan data 10 illustrated in FIG. 3 into scan data corresponding to the impression scan data 20, as illustrated in FIG. 4, a normal of the oral scan data 10 is changed, that is, a model of the oral scan data 10 becomes a negative model. Accordingly, the oral scan data 10 is represented as reverse data 10' of the oral scan data, and the oral scan data 10 and the impression scan data 20 are compared and merged in a one-to-one manner.

Next, as illustrated in FIG. 5, scan data of a selection area A among the converted impression scan data 20 is partially obtained. The obtained scan data is applied by replacing the oral scan data 10 having the changed impression mode (S230). In this case, information on the scan data of the selection area A to be alternatively applied further includes scan data information of partial areas neighboring scan data information of the selection area A, that is, before and after the selection area A. Accordingly, the information on the scan data of the selection area A can be alternatively applied to an accurate area because the selection area A is automatically simulated before the scan data of the selection area A is alternatively applied to the oral scan data 10 of the selection area A. In this case, the selection area A corresponds to a margin line M in which measurement is difficult because the margin line is covered with a gum tissue in an environment within a mouth, a post area P formed of an engraved portion, or a metal area filled with amalgam or resin. For this reason, the selection area A needs to be replaced with information on a corresponding area of the impression scan data 20 because accurate scan data of such a portion cannot be secured.

Finally, as illustrated in FIG. 6, a mode of the oral scan data 10 having the state in which the selection area A has been alternatively applied and having the changed impression mode is changed into an oral mode again, that is, an initial mode (S240). As illustrated in FIG. 7, the final scan datum 30 is obtained, and the process is terminated (S300). Accordingly, in the present embodiment, the one final scan datum 30 in which the oral scan data 10 and the impression scan data 20 have been merged can be obtained because a process of aligning the impression scan data 20 with the oral scan data 10 is performed in real time by continuously changing a mode of the selection area A into the impression mode and the oral mode in a process of obtaining the impression scan data 20 after obtaining the oral scan data 10. More specifically, data of the selection area A among the oral scan data 10 needs to be supplemented. The oral scan data 10 is converted into the reverse data 10' of the oral scan data in the impression mode. The impression scan data 20 in the selection area A may be applied to replace some of the reverse data 10' of the oral scan data. Accordingly, the one final scan datum 30 merged by changing the impression mode into the oral mode again can be obtained.

Accordingly, in the present embodiment, the oral scan data 10 and the impression scan data 20 are independently obtained. The obtained data is divided into a portion to be maintained in the oral scan data 10 and a portion to be replaced with the impression scan data 20, that is, the selection area A. The portions are partially aligned and merged. Accordingly, when scan data is obtained by the oral scanner, a portion not clearly represented using only the oral scan data 10 can be represented in the same level as an actual oral object by selectively supplementing the portion by using the impression scan data 20. As a result, the accurate final scan datum 30 which may be used in a task for designing the oral object can be effectively secured.

The final scan datum 30 obtained as described above may be output in the form of a file having a common format without a separate additional task. The final scan datum 30 may be transmitted to a design program, so that prosthetics for an oral object having the same level as an actual mouth can be fabricated (S400).

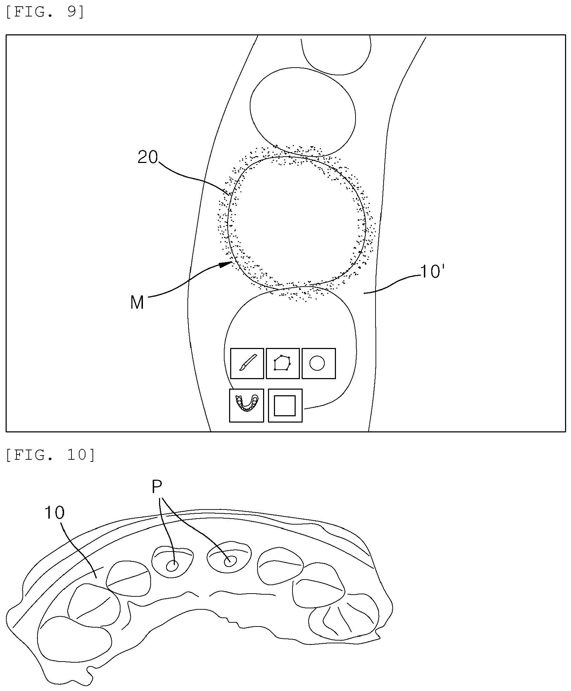

Hereinafter, FIG. 8 is a diagram illustrating the indication of a margin line in a combined oral and impression scanning method according to a second embodiment of the present disclosure. FIG. 9 is a diagram illustrating the replacement and application of a margin line in the combined oral and impression scanning method according to the second embodiment of the present disclosure.

As illustrated in FIG. 8, in the impression mode change step S220, as a result of the scanning of the inside of a mouth through the oral scanner, when it is determined that data is unclearly obtained because the indication of the margin line M corresponding to a boundary between gums in the oral scan data 10 is covered, the margin line M may be represented through scanning in the state in which an impression material enabling the impression scan data 20 to be obtained is filled between the gums and teeth. That is, it is difficult to obtain the margin line M by using the oral scanner. The margin line M may be obtained using the impression material filled between the gums and the teeth. As illustrated in FIG. 9, the obtained margin line M, more specifically, the margin line M indicated in the impression scan data 20 may be applied to the oral scan data 10 and indicated when the final scan datum 30 is obtained in the final scan data acquisition step S300.

Accordingly, in the present embodiment, the margin line M whose measurement is difficult because the margin line is covered with a gum tissue in a mouth is applied to the oral scan data 10 by using an impression material and indicated in the final scan datum 30. Accordingly, prosthetics having high precision can be fabricated because data having more abundant and clearer information is delivered to a design program.

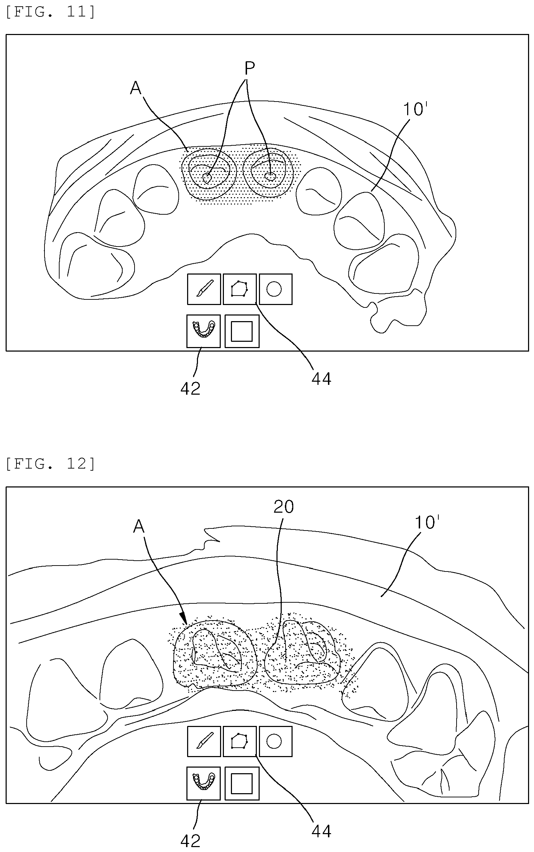

Hereinafter, FIG. 10 is a diagram illustrating a post area P in a combined oral and impression scanning method according to a third embodiment of the present disclosure. FIG. 11 is a diagram illustrating the state in which a selection area for the post area P is indicated in the combined oral and impression scanning method according to the third embodiment of the present disclosure. FIG. 12 is a diagram illustrating that embossing scan data is applied to the post area in the combined oral and impression scanning method according to the third embodiment of the present disclosure.

As illustrated in FIG. 10, as a result of the scanning of an oral object, when it is determined that the post area P is included, a mode of the post area P may be changed into the impression mode and applied by replacing previously stored impression scan data.

Such a post area scan step may be performed in an order to be described later. In general, in the case of a post treatment process, there is a difficulty in a process of obtaining scan data by using the oral scanner because the post area P has an engraved form and has a large depth. For this reason, first, the post area P is indicated with respect to an oral object. That is, as illustrated in FIG. 10, the indicated post area P is indicated in the state in which the post area P has been perforated due to its depth. In order to obtain scan data of the post area P, the oral scan data 10 of the post area P is selectively obtained and indicated. Thereafter, as illustrated in FIG. 11, a mode of the oral object including the post area P is changed into the impression mode in order to obtain the impression scan data 20. In this case, the step of determining whether to store the impression scan data is performed by confirming whether the presence of the post area P corresponds to the preset determination condition after the step of storing the oral scan data 10. Only a portion corresponding to a portion of the oral scan data corresponding to the determination condition, among the impression scan data, may be selectively scanned and stored.

As a result, the impression scan data 20 of the post area P can be obtained by changing the mode of the oral object into the impression mode. As illustrated in FIG. 12, if the post area P is present after embossing scan data is applied by partially replacing the impression scan data 20 with the embossing scan data, the post area P may also be incorporated into the final scan datum 30 when the impression mode is changed into the oral mode again in the final scan data acquisition step S300.

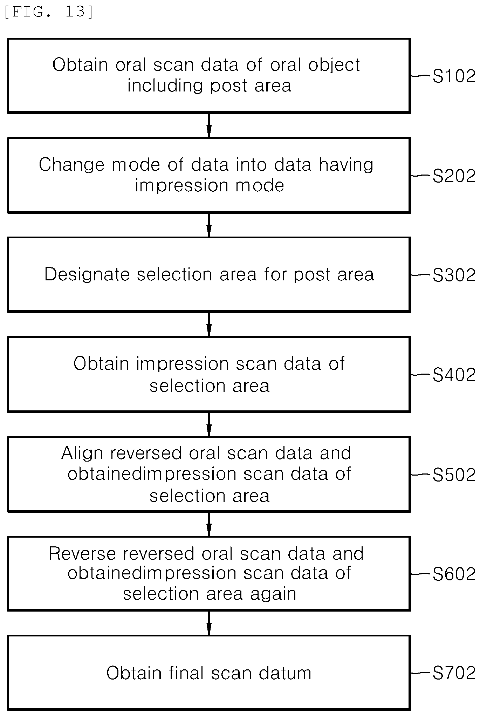

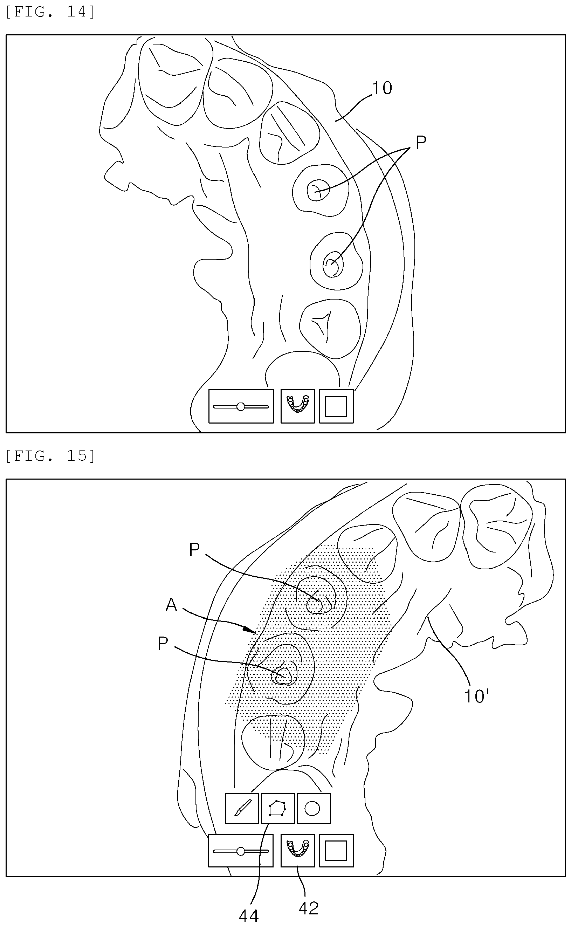

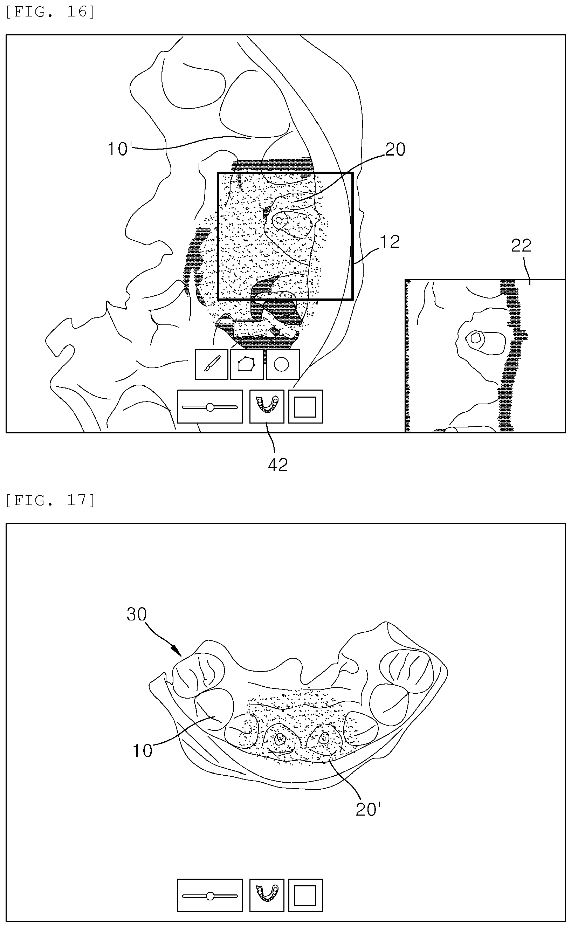

FIG. 13 is a diagram sequentially illustrating a combined oral and impression scanning method according to a fourth embodiment of the present disclosure. FIG. 14 is a diagram illustrating a post area P in the combined oral and impression scanning method according to the fourth embodiment of the present disclosure. FIG. 15 is a diagram illustrating the state in which a selection area A for the post area is indicated in the combined oral and impression scanning method according to the fourth embodiment of the present disclosure. FIG. 16 is a diagram illustrating that embossing scan data is applied to the post area P in the combined oral and impression scanning method according to the fourth embodiment of the present disclosure. FIG. 17 is a diagram illustrating the final scan data obtained using the combined oral and impression scanning method according to the fourth embodiment of the present disclosure.

First, as illustrated in FIG. 13, in the combined oral and impression scanning method according to the fourth embodiment of the present disclosure, an oral scan data acquisition step S102 of obtaining oral scan data 10 by scanning an oral object through the scanner is performed. In this case, the oral object includes a post and core task area (hereinafter referred to as a post area P) for a crown or implant task. More specifically, the oral object has the post area P as a part of the object. From FIG. 14, it may be seen that the oral scan data 10 appears and the post area P has been incompletely scanned.

In order to supplement data by obtaining additional information on the post area P, data of the post area P may be supplemented in the impression mode. That is, this means that data reversed by changing a mode of the data having the oral mode into data having the impression mode (S202) is supplemented using newly obtained data having an impression form. As illustrated in FIG. 15, the obtained oral scan data 10 may be represented as reverse data 10' of the oral scan data by clicking on an impression mode change button 42. Thereafter, the incompletely scanned post area P may be designated as a selection area A (S302). The selection area A may be designated by clicking on an area selection button 44 disposed close to the impression mode change button 42 and including the post area P among the reverse data 10' of the oral scan data. The selection area A is shaded, and impression scan data 20 corresponding to a corresponding portion may be obtained.

When the selection area A is designated, impression scan data 20 corresponding to the selection area A may be obtained by additional scanning using the scanner (S402). Meanwhile, the impression scan data 20 is obtained by scanning an impression model, that is, a frame of an oral model, through the scanner. When the impression model is scanned, a current state of the scanning is displayed on a scan screen 22, and a process of obtaining corresponding data is displayed in the scan area 12. The post area P obtained as the impression scan data 20 actually has an engraved form. However, in the impression mode, the post area P may also be obtained in an embossing form because the oral scan data 10 is reversed. As described above, the obtained impression scan data 20 is aligned with a portion corresponding to the selection area A among the reverse data 10' of the oral scan data (S502). As the impression scan data 20 is aligned, the impression scan data 20 can supplement an insufficient scan portion of the oral scan data 10. Meanwhile, the oral scan data 10 of the selection area A including the post area P may be applied to be replaced with relatively precise impression scan data 20.

As illustrated in FIG. 17, after the data alignment S502 is performed, the impression mode may be released by clicking on the impression mode change button 42. That is, the scan mode may return from the impression mode to the oral mode. The data is reversed again by releasing the impression mode (S602). More specifically, the reverse data 10' of the oral scan data is indicated as the oral scan data 10 again. The impression scan data 20 is indicated as reverse data 20' of the impression scan data. Meanwhile, since the impression scan data 20 is a negative model, the reverse data 20' of the impression scan data may be used to obtain the final scan datum 30. Meanwhile, the impression scan data 20 and the reverse data 20' of the impression scan data may be formed to have a normal in which the impression scan data 20 and the reverse data 20' face each other.

As illustrated in FIG. 17, a data value of the selection area A of the oral scan data 10 may be changed due to the impression scan data 20. For example, the change of the data value may mean that a data value in the existing selection area A is replaced or merged with or supplemented by a data value of the impression scan data 20. Preferably, impression scan data obtained by performing an impression scan may replace the reverse data 10' of oral scan data corresponding to a selection area (in particular, an area including a post area). Furthermore, one final scan datum 30 is obtained by combining the oral scan data 10 and the impression scan data 20 (S702). The combined scan data has high reliability, so that precise prosthetics can be fabricated.

Meanwhile, unnecessary data can be prevented from being accumulated and the final scan datum 30 having high reliability can also be obtained by obtaining the impression scan data 20 of only the selection area A corresponding to the post area P.

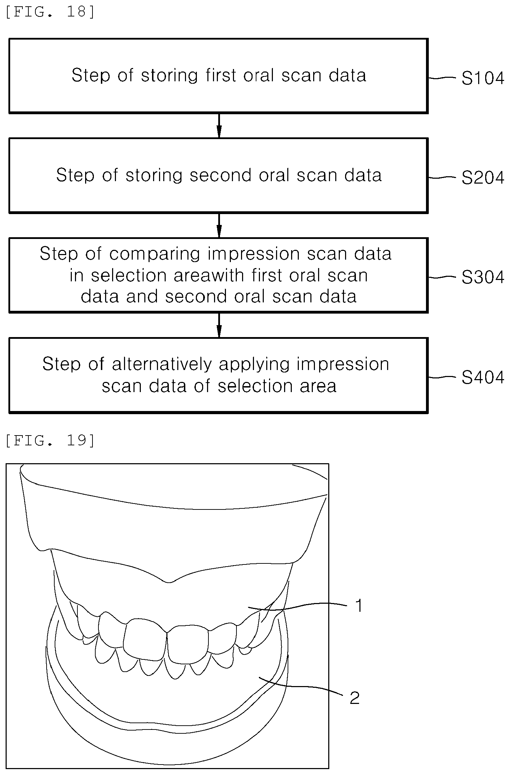

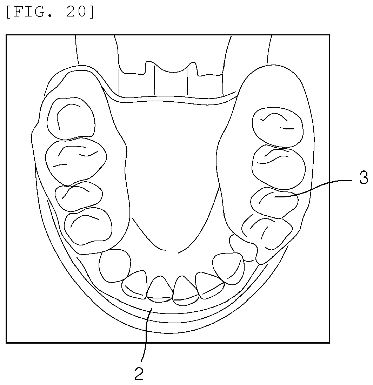

Hereinafter, FIG. 18 is a diagram sequentially illustrating a combined oral and impression scanning method according to a fifth embodiment of the present disclosure. FIG. 19 is a diagram illustrating the state in which an upper jaw and a lower jaw face each other in the combined oral and impression scanning method according to the fifth embodiment of the present disclosure. FIG. 20 is a diagram illustrating a covered state of an impression material in the combined oral and impression scanning method according to the fifth embodiment of the present disclosure.

In general, in scanning an occlusion state of an oral object, a method of directly scanning the occlusion state in a mouth may have a difficulty in terms of its precision. Accordingly, scan data of the occlusion state may be precisely secured by merging oral scan data and impression scan data. To this end, as illustrated in FIG. 18 and FIG. 19, a method of securing accurate scan data of the occlusion state is described as follows.

First, as illustrated in FIG. 18, first oral scan data is stored by scanning an outer surface of an oral object in the state in which the upper jaw 1 and lower jaw 2 of the oral object face each other in a way to be occluded (S104). Furthermore, second oral scan data is stored by scanning the inside of the oral object in the state in which the upper jaw 1 and the lower jaw 2 are widened in the state in which the upper jaw 1 and the lower jaw 2 face each other (S204).

Thereafter, impression scan data of a selection area of the oral object including the occlusion state of the upper jaw 1 and the lower jaw 2 is obtained and compared with the first oral scan data and the second oral scan data (S304). That is, as illustrated in FIG. 18 and FIG. 20, for example, when molar portions on both side of the oral object are covered with an impression material 3 and the upper jaw 1 and the lower jaw 2 face each other, the states in which the upper jaw 1 and the lower jaw 2 are aligned with upper and lower parts of the impression material 3, respectively, are indicated. Furthermore, a difference between the locations of the upper jaw 1 and the lower jaw 2, that is, the occlusion state of the upper jaw 1 and the lower jaw 2, may also be indicated. Accordingly, an accurate occlusion state of the oral object can be checked by scanning information on the indicated impression material 3 and comparing corresponding scan data with the previously stored first oral scan data and second oral scan data.

Finally, impression scan data corresponding to corresponding locations, that is, the molar portions on both sides described as an example, among the first oral scan data and the second oral scan data, are partially replaced (S404), so that the final scan data including information on an accurate occlusion state of the oral object is obtained. The reason for this is that since the impression scan data obtained using the impression material is more precise than the scan data obtained by scanning the inside and outside of the oral object through the oral scanner, the first oral scan data and the second oral scan data can be applied by partially replacing the impression scan data and accurate occlusion state information can be obtained.

The present disclosure has an effect in that elaborate prosthetics can be fabricated using obtained digital data in a way to obtain one final scan datum merged by aligning impression scan data with oral scan data in real time in a process of obtaining the impression scan data after obtaining the oral scan data by using the oral scanner.

Furthermore, the present disclosure has an effect in that more accurate digital data can be obtained by expressing a margin line in a process of obtaining an impression when it is difficult to measure the margin line because the margin line is covered with a gum tissue in an environment within a mouth.

Meanwhile, the present disclosure has an effect in that it can solve a conventional problem in which it is difficult or impossible to obtain data by using the oral scanner due to the depth of a post area having an engraved form, in such a way to enable a portion whose scanning is impossible to be scanned by obtaining, in an embossing form, the post area actually having the engraved form by obtaining impression scan data.

Furthermore, the present disclosure has an effect in that a teeth portion whose accurate scanning is difficult due to a material characteristic can be scanned by separately obtaining an impression of the teeth portion covered with a crown made of metal and using such impression scan data for occlusion and alignment necessary to obtain the final scan data.

The above description is merely a description of the technical spirit of the present disclosure, and those skilled in the art may change and modify the present disclosure in various ways without departing from the essential characteristic of the present disclosure.

The range of protection of the present disclosure should be construed based on the following claims.

### [Industrial Applicability]

The present disclosure provides the combined oral and impression scanning system and method for obtaining one final scan datum merged by aligning impression scan data with oral scan data in a process of obtaining the impression scan data after obtaining the oral scan data.

## Claims

1. A combined oral and impression scanning system comprising:
an oral scanner (100) configured to scan an oral object;
a storage unit (200) configured to store oral scan data (10) of the oral object which represents an oral model or an inside of a mouth of a patient obtained from the oral scanner (100) and selectively store impression scan data (20) of the oral object which represents an impression model that is a frame of the oral model according to a preset determination condition with respect to the oral object;
a determination unit (300) configured to determine whether the oral scan data (10) corresponds to the determination condition and guide selective storage of the impression scan data (20); and
a control unit (400) configured to control a selection area (A) corresponding to the determination condition, among the impression scan data (20), to be partially applied to a corresponding area of the oral scan data (10) to obtain a final scan data (30).

2. The combined oral and impression scanning system according to claim 1, wherein the determination condition comprises at least one of a plurality of conditions, comprising unclear indication of a margin line (M), presence of a post area (P) or presence of an area comprising metal in the oral scan data (10).

3. The combined oral and impression scanning system according to claim 1 or 2, wherein the control unit (400) comprises a simulation unit (410) configured to receive, from the storage unit (200), information on areas neighboring the selection area (A) and to automatically simulate the selection area (A) before the selection area (A) is applied to be replaced.

4. A combined oral and impression scanning method comprising:
an oral scan data storage step (S100) of storing oral scan data (10) to a storage unit (200) by scanning an oral object which represents an oral model or an inside of a mouth of a patient with an oral scanner (100);
an impression mode change step (S220) of changing a mode of the oral scan data (10) into an impression mode by determining the oral scan data (10) so that the oral scan data (10) corresponds to selectively stored impression scan data (20) which represents an impression model that is a frame of the oral model;
a selection area replacement application step (S230) of partially obtaining scan data of a selection area (A) among the impression scan data (20) by the oral scanner (100) and applying the impression scan data to the oral scan data (10) having the changed impression mode by a control unit (400); and
a final scan datum acquisition step (S300) of changing again, into an oral mode which is an initial mode, the impression mode of the oral scan data (10) to which the impression scan data (20) has been applied in the selection area (A) and obtaining a final scan data (30) by the control unit (400) .

5. The combined oral and impression scanning method according to claim 4, further comprising an impression scan data storage determination step (S200) of selectively storing the impression scan data (20) depending on whether the oral scan data (10) comprises a preset determination condition after the oral scan data storage step (S100).

6. The combined oral and impression scanning method according to claim 5, wherein the impression scan data (20) comprises data stored by selectively scanning only a portion corresponding to a portion of oral scan data (10) corresponding to the determination condition in an impression model.

7. The combined oral and impression scanning method according to any one of claims 4 to 6, wherein:
the impression mode change step (S220) comprises indicating a margin line (M) in the impression scan data (20) by using an impression material when it is determined that indication of the margin line (M) is unclear in the oral scan data (10) in the oral scan data storage step (S100), and
the margin line (M) is applied to the oral scan data (10) and indicated when the final scan data (30) is obtained.

8. The combined oral and impression scanning method according to any one of claims 4 to 7, wherein the oral scan data storage step (S100) comprises a post area scan step of converting a mode of the post area (P) into an impression mode when it is determined that the selection area (A) is a post area (P) by scanning the oral object and applying the selection area (A) by replacing previously stored impression scan data (20).

9. The combined oral and impression scanning method according to claim 8, wherein the post area scan step comprises:
a display step of displaying the post area (P);
an impression mode change step of selectively obtaining oral scan data (10) of the post area (P), converting a mode of the oral scan data (10) into the impression mode, and obtaining impression scan data (20); and
an embossing scan data application step of obtaining embossing scan data of the post area (P) by changing the impression mode and applying the embossing scan data to the impression scan data (20) by partially replacing the impression scan data (20).

## Patentansprüche

1. Kombiniertes Mund- und Abdruck-Scansystem, umfassend:
einen Mundscanner (100), der zum Scannen eines Mundobjekts konfiguriert ist;
eine Speichereinheit (200), die konfiguriert ist, um Mundscandaten (10) des Mundobjekts zu speichern, das ein Mundmodell oder ein Inneres eines Mundes eines Patienten darstellt, das von dem Mundscanner (100) erhalten wurde, und selektiv Abdruckscandaten (20) des Mundobjekts zu speichern, die ein Abdruckmodell darstellen, das ein Rahmen des Mundmodells gemäß einer voreingestellten Bestimmungsbedingung in Bezug auf das Mundobjekt ist;
eine Bestimmungseinheit (300), die konfiguriert ist, um zu bestimmen, ob die Mundscandaten (10) der Bestimmungsbedingung entsprechen und die selektive Speicherung der Abdruckscandaten (20) zu führen; und
eine Steuereinheit (400), die konfiguriert ist, um einen der Bestimmungsbedingung entsprechenden Auswahlbereich (A) unter den Abdruckscandaten (20) zu steuern, der teilweise auf einen entsprechenden Bereich der Mundscandaten (10) anzuwenden ist, um endgültige Scandaten (30) zu erhalten.

2. Kombiniertes Mund- und Abdruck-Scansystem nach Anspruch 1, wobei die Bestimmungsbedingung mindestens eine von einer Vielzahl von Bedingungen bestimmt, umfassend die unklare Anzeige einer Randlinie (M), das Vorhandensein eines Postbereichs (P) oder das Vorhandensein eines Metall enthaltenden Bereichs in den Mundscandaten (10).

3. Kombiniertes Mund- und Abdruck-Scansystem nach Anspruch 1 oder 2, wobei die Steuereinheit (400) eine Simulationseinheit (410) umfasst, die so konfiguriert ist, dass sie von der Speichereinheit (200) Informationen über Bereiche, die an den Auswahlbereich (A) angrenzen, empfängt und den Auswahlbereich (A) automatisch simuliert, bevor der Auswahlbereich (A) zum Ersetzen angewendet wird.

4. Kombiniertes Mund- und Abdruck-Scanverfahren, umfassend:
einen Mundscandaten-Speicherschritt (S100) zum Speichern von Mundscandaten (10) in einer Speichereinheit (200) durch Scannen eines Mundobjekts, das ein Mundmodell oder das Innere eines Mundes eines Patienten darstellt, mit einem Mundscanner (100);
einen Abdruckmodus-Änderungsschritt (S220) zum Ändern eines Modus der Mundscandaten (10) in einen Abdruckmodus durch Bestimmen der Mundscandaten (10), so dass die Mundscandaten (10) selektiv gespeicherten Abdruckscandaten (20) entsprechen, die ein Abdruckmodell darstellen, das ein Rahmen des Mundmodells ist;
einen Auswahlbereich-Ersatzanwendungsschritt (S230) zum teilweise Erhalten von Scandaten eines Auswahlbereichs (A) unter den Abdruckscandaten (20) durch den Mundscanner (100) und Anwenden der Abdruckscandaten auf die Mundscandaten (10) mit dem geänderten Abdruckmodus durch eine Steuereinheit (400); und
einen finalen Scandaten-Erfassungsschritt (S300) zum erneuten Umstellen auf einen Mundmodus, der ein anfänglicher Modus ist, der den Abdruckmodus der Mundscandaten (10) betrifft, auf die die Abdruckscandaten (20) im Auswahlbereich (A) angewendet wurden, und Erhalten von finalen Scandaten (30) durch die Steuereinheit (400).

5. Kombiniertes Mund- und Abdruck-Scanverfahren nach Anspruch 4, umfassend einen Abdruckscandaten-Speicherbestimmungsschritt (S200) zum selektiv Speichern der Abdruckdaten (20), je nachdem, ob die Mundscandaten (10) eine voreingestellte Bestimmungsbedingung nach dem Mundscandaten-Speicherschritt (S100) umfassen.

6. Kombiniertes Mund- und Abdruck-Scanverfahren nach Anspruch 5, wobei die Abdruckscandaten (20) Daten umfassen, die durch selektives Scannen nur eines Teils gespeichert wurden, der einem Teil der Mundscandaten (10) entspricht, der der Bestimmungsbedingung in einem Abdruckmodell entspricht.

7. Kombiniertes Mund- und Abdruck-Scanverfahren nach einem der Ansprüche 4 bis 6, wobei:
der Abdruckmodus-Änderungsschritt (S220) das Anzeigen einer Randlinie (M) in den Abdruckscandaten (20) unter Verwendung eines Abdruckmaterials umfasst, wenn bestimmt wird, dass die Anzeige der Randlinie (M) in den Mundscandaten (10) in dem Mundscandaten-Speicherschritt (S100) unklar ist, und
die Randlinie (M) auf die Mundscandaten (10) angewendet und angezeigt wird, wenn die finalen Scandaten (30) erhalten werden.

8. Kombiniertes Mund- und Abdruck-Scanverfahren nach einem der Ansprüche 4 bis 7, wobei der Mundscandaten-Speicherschritt (S100) einen Postbereich-Scanschritt umfasst zum Umwandeln eines Modus des Postbereiches (P) in einen Abdruckmodus, wenn durch Scannen des Mundobjekts bestimmt wird, dass der Auswahlbereich (A) ein Postbereich (P) ist, und Anwenden des Auswahlbereichs (A) durch Ersetzen zuvor gespeicherter Abdruckscandaten (20).

9. Kombiniertes Mund- und Abdruck-Scanverfahren nach Anspruch 8, wobei der Postbereich-Scanschritt umfasst:
einen Anzeigeschritt zum Anzeigen des Postbereichs (P);
einen Abdruckmodus-Änderungsschritt zum selektiv Erhalten der Mundscandaten (10) des Postbereichs (P), zum Umwandeln eines Modus der Mundscandaten (10) in den Abdruckmodus und zum Erhalten der Abdruckscandaten (20); und
einen Prägescandaten-Anwendungsschritt zum Erhalten der Prägescandaten des Postbereichs (P) durch Ändern des Abdruckmodus und zum Anwenden der Prägescandaten auf die Abdruckscandaten (20), indem die Abdruckscandaten (20) teilweise ersetzt werden.

## Revendications

1. Système de balayage d'empreinte et oral combiné, comprenant :
un scanner oral (100) configuré pour balayer un objet oral ;
une unité de stockage (200) configurée pour stocker des données de balayage oral (10) de l'objet oral, lequel représente un modèle oral ou un intérieur d'une bouche d'un patient obtenu à partir du scanner oral (100), et stocker de manière sélective des données de balayage d'empreinte (20) de l'objet oral, lequel représente un modèle d'empreinte, lequel est un cadre du modèle oral conformément à une condition de détermination préréglée par rapport à l'objet oral ;
une unité de détermination (300) configurée pour déterminer si oui ou non les données de balayage oral (10) correspondent à la condition de détermination, et guider un stockage sélectif des données de balayage d'empreinte (20), et
une unité de commande (400) configurée pour commander une zone de sélection (A) correspondant à la condition de détermination, parmi les données de balayage d'empreinte (20), devant être appliquée partiellement à une zone correspondante des données de balayage oral (10) afin d'obtenir des données de balayage final (30).

2. Système de balayage d'empreinte et oral combiné selon la revendication 1, dans lequel la condition de détermination comprend au moins une condition parmi une pluralité de conditions, comprenant une indication peu claire d'une ligne de marge (M), une présence d'une zone de tenon (P), ou une présence d'une zone comprenant du métal dans les données de balayage oral (10).

3. Système de balayage d'empreinte et oral combiné selon la revendication 1 ou 2, dans lequel l'unité de commande (400) comprend une unité de simulation (410) configurée pour recevoir, à partir de l'unité de stockage (200), des informations sur des zones avoisinantes de la zone de sélection (A), et pour simuler automatiquement la zone de sélection (A), avant que la zone de sélection (A) ne soit appliquée pour remplacement.

4. Procédé de balayage d'empreinte et oral combiné, comprenant les étapes suivantes :
une étape de stockage des données de balayage oral (S100) pour stocker des données de balayage oral (10) dans une unité de stockage (200), en balayant un objet oral, lequel représente un modèle oral ou un intérieur d'une bouche d'un patient avec un scanner oral (100) ;
une étape de modification de mode d'empreinte (S220) pour modifier un mode des données de balayage oral (10) en un mode d'empreinte en déterminant les données de balayage oral (10) de sorte que les données de balayage oral (10) correspondent à des données de balayage d'empreinte stockées de manière sélective (20), lesquelles représentent un modèle d'empreinte, lequel est un cadre du modèle oral ;
une étape d'application de remplacement de zone de sélection (S230) pour obtenir partiellement des données de balayage d'une zone de sélection (A) parmi les données de balayage d'empreinte (20) par le scanner oral (100), et appliquer les données de balayage d'empreinte aux données de balayage oral (10) présentant le mode d'empreinte modifié par une unité de commande (400), et
une étape d'acquisition d'origine de balayage final (S300) pour modifier à nouveau, vers un mode oral, lequel est un mode initial, le mode d'empreinte des données de balayage oral (10) auquel les données de balayage d'empreinte (20) ont été appliquées dans la zone de sélection (A), et obtenir des données de balayage final (30) par l'unité de commande (400).

5. Procédé de balayage d'empreinte et oral combiné selon la revendication 4, comprenant en outre une étape de détermination de stockage de données de balayage d'empreinte (S200) pour stocker de manière sélective les données de balayage d'empreinte (20) en fonction de si oui ou non les données de balayage oral (10) comprennent une condition de détermination préréglée après l'étape de stockage de données de balayage oral (S100).

6. Procédé de balayage d'empreinte et oral combiné selon la revendication 5, dans lequel les données de balayage d'empreinte (20) comprennent des données stockées en balayant de manière sélective uniquement une portion correspondant à une portion de données de balayage oral (10) correspondant à la condition de détermination dans un modèle d'empreinte.

7. Procédé de balayage d'empreinte et oral combiné selon l'une quelconque des revendications 4 à 6, dans lequel :
l'étape de modification de mode d'empreinte (S220) comprend l'étape pour indiquer une ligne de marge (M) dans les données de balayage d'empreinte (20) à l'aide d'un matériau d'empreinte lorsqu'il est déterminé que l'indication de la ligne de marge (M) n'est pas claire dans les données de balayage oral (10) lors de l'étape de stockage de données de balayage oral (S100), et
la ligne de marge (M) est appliquée aux données de balayage oral (10) et indiquée lorsque les données de balayage final (30) sont obtenues.

8. Procédé de balayage d'empreinte et oral combiné selon l'une quelconque des revendications 4 à 7, dans lequel l'étape de stockage de données de balayage oral (S100) comprend une étape de balayage de zone de tenon pour convertir un mode de la zone de tenon (P) en un mode d'empreinte lorsqu'il est déterminé que la zone de sélection (A) est une zone de tenon (P) en balayant l'objet oral et en appliquant la zone de sélection (A) en remplaçant les données de balayage d'empreinte stockées précédemment (20).

9. Procédé de balayage d'empreinte et oral combiné selon la revendication 8, dans lequel l'étape de balayage de zone de tenon comprend :
une étape d'affichage pour afficher la zone de tenon (P) ;
une étape de modification de mode d'empreinte pour obtenir de manière sélective des données de balayage oral (10) de la zone de tenon (P), convertir un mode des données de balayage oral (10) en mode d'impression, et obtenir des données de balayage d'impression (20), et
une étape d'application de données de balayage de gaufrage pour obtenir des données de balayage de gaufrage de la zone de tenon (P) en modifiant le mode d'empreinte, et en appliquant les données de balayage de gaufrage aux données de balayage d'empreinte (20) en remplaçant partiellement les données de balayage d'empreinte (20).
